# EUROPEAN PATENT APPLICATION

(11) **EP 4 306 040 A1**
(43) Date of publication of application: **17.01.2024**
(21) Application number: 22184209.9
(22) Date of filing: 11.07.2022
(51) Int. Cl.: A61B 5/00

(54) **A PROBE DEVICE FOR INTERFACING WITH CELLS SUCH AS NEURONS AND ASSOCIATED DEVICES, METHODS, AND USE**

(71) Applicant: Atlas Neuroengineering BV, 3000 Leuven (BE)
(72) Inventor: SUN, Jyh-Jang, 3010 Kessel-Lo (BE); KLOOSTERMAN, Fabian, Washington DC 20001 (US); KAO, Kuo-Hsing, 701 Tainan City (TW); VAN DAAL, Rik, 3000 Leuven (BE); AARTS, Arno, 3000 Leuven (BE)
(74) Representative: IPLodge bv

(57) **Abstract**

A probe device for interfacing with cells, as for instance neurons, preferably individual neurons, in a brain tissue, comprising a support structure having a longitudinal axis and set of neuron interfacing electrodes arranged on the support structure, wherein the set of neuron interfacing electrodes are arranged and adapted to be movable between a first, insertion configuration and a second extended configuration, the first configuration being different from the second configuration, wherein the first configuration is adapted for reaching a predetermined target area in the brain while creating a minimal damage to the brain, wherein the second configuration corresponding to a predetermined regular 3-dimensional grid configuration, the predetermined regular 3-dimensional grid configuration corresponding to a cubic, face centered cubic (fcc), body centered cubic (bcc), hexagonal or tetrahedron grid configuration.

## Description

### Technical field

The present disclosure relates to the field of medical probes such as neural probes.

### Background art

Thorough understanding about brain functioning is a global scientific challenge. Neuro-electrophysiological knowledge is extended by increasing the large-scale recording capability of isolated single-unit activities. For this, an electrode array in 3D seems to give the best yield of units, due to their relatively larger monitoring volume and relatively increased monitoring volume. An increased monitoring volume can improve neuron localization and increases the number of detected neurons which leads to robust spike sorting.

Most state-of-the-art neural probes comprising of a single shaft or several shafts that contain(s) one or several electrodes only sample in one-dimensional (1D) or two-dimensional (2D) space. They are limited to measurements along a small cylindrical volume around the probe shaft, which is not sufficient to measure all neurons within a cortical column. Although three-dimensional (3D) rigid silicon neural probes (by stacking several 2D silicon probes) have been commercialized, they cause significant tissue damage and are difficult to insert into the brain. The high density of penetrating probe shafts results in a large tissue dimpling during implantation which results in cell loss. Also, those 3D probe arrays have limited depth and cannot reach deep brain areas that are beyond 10mm of depth.

The exist a need for neural probes with better neuron localization and increased neuron identification with reduced tissue damage using a limited number of electrodes, which can lead to new therapeutic insights in solving brain related diseases and disorders.

### Summary of the disclosure

It is an objective of the present disclosure to provide a probe device for interfacing with, preferably individual cells or a small set of cells (e.g. 2, 3 , 4 , 5 cells), preferably neuron cells, according to claim 1, a related composite probe device, a use of the probe device or probe composite device, a method for determining individual neuron positions in brain tissue, a method for measuring neuron activity in brain tissue, a method for stimulating neurons in brain tissue, and a method for manufacturing the probe device for interfacing with neurons in a brain.

In a first aspect of the present disclosure, a probe device for interfacing with cells, preferably neuron cells, preferably individual cells or neurons, alternatively with a plurality of cells or neurons in a brain tissue, such as a neural probe, has been disclosed, comprising a support structure having a longitudinal axis and a set of cell or neuron interfacing devices arranged on the support structure. The set of cell or neuron interfacing devices is arranged and adapted to be movable between a first, insertion configuration and a second extended configuration, the first configuration being different from the second configuration. The first configuration is preferably adapted for reaching a predetermined target area in the brain while creating a minimal damage to the brain. The second configuration preferably corresponds to a predetermined regular 3-dimensional grid configuration being one of a cubic, a face centered cubic (fcc), body centered cubic (bcc), tetrahedron or hexagonal grid configuration.

It is an advantage that a neural probe comprising cell/neuron interfacing devices in any of these configurations allow a much better individual cell/neuron identification when compared to for instance cell/neuron interfacing devices in a random configuration, for the same amount of cell/neuron interfacing devices per volume.

According to preferred embodiments, the probe device is configured such that the electrode sensing radius is within the range of 75 micron to 150 micron, and such that the electrode pitch is within the range of 45 micron to 150 micron.

Herein, the electrode pitch is the minimal electrode distance for the respective predetermined regular 3D grid configuration.

Preferably, the neuron interfacing devices are electrodes. Alternatively, the neuron interfacing devices are optical interaction devices, which comprise light sources (such as e.g. LED's) and/or optical sensors. According to preferred embodiments, the neuron interfacing devices are electrodes, and adjacent to some or all of the electrodes, a corresponding optical interaction device can be provided. The latter allows to not only identify individual neurons and provide electrical stimulation or readout thereof, but also to stimulate the identified neurons by an optical beam or signal. For instance, different types of neurons can be labelled such that each type of neurons becomes sensitive, i.e. active and silenced, to a specific type of light wave, such as for instance a specific wavelength, and thus to a specific type of LED.

According to preferred embodiments, the support structure comprises a flexible or stretchable material such as a flexible biocompatible polymer. The support structures are stiff enough during the insertion configuration in order to penetrate the tissue. Preferably, the flexible biocompatible polymer comprises polyimide, parylene, epoxy, PEEK, Ultem, SU-8, PDMS, silicone. Alternatively dissolvable materials can be used that are stiff during implantation and dissolve over time once implanted, e.g. in the environment of brain tissue.

According to preferred embodiments, the support structure generally defines a longitudinal probe shape defining a circumferential wall comprising a cylinder mantle portion describing an angular section of a cylinder surface, and the neuron interfacing devices are abutting the cylinder mantle portion, when in the first configuration.

The support structure can preferably comprise a circular cross-section in a direction perpendicular on its longitudinal axis. Such a circular cross-sectional support structure provides an advantage over typically rectangular cross-sectional silicon-based probes since substantially less tissue damage is generated during insertion of the probe into brain tissue. Tissue damage results in scar tissue to be formed, further jeopardizing the recording and stimulation function of the neural probe.

According to preferred embodiments, the support structure comprises a longitudinal central portion and a set of a plurality of at least 1 (e.g.1) (or at least 2 (e.g. 2), or at least 3 (e.g. 3), or at least 4 (e.g. 4)) flexible arm(s), each arm generally extending from a respective first end at a central portion of the support structure to a second end being a freestanding distal end, for instance forming a 3D grid. Preferably, each arm comprises at least one neuron interfacing device, wherein the arms are adapted and arranged for positioning the neuron interfacing devices in the predetermined regular grid configuration by bending radially outwards according to a predetermined extent, when in the second configuration (corresponding to a second configuration of the arms). Preferably, each of the flexible arms has a pointed tip suitable for being inserted into brain tissue.

According to alternative preferred embodiments, the support structure comprises a longitudinal central portion and a set of a plurality of flexible arms, each arm generally extending from a respective first end at a central portion of the support structure to a second end being a freestanding distal end. Preferably, each arm comprises at least one neuron interfacing device, such as an electrode, and the central portion comprises one or more neuron interfacing devices, such as electrodes, and the central portion and the flexible arms are adapted and arranged for positioning the neuron interfacing devices in the predetermined regular grid configuration by bending the flexible arms radially outwards according to a predetermined extent, when in the second configuration.

According to preferred embodiments, the predetermined extent is determined by a predetermined internal stress in the arms causing a radially outward bias of the distal end with respect to the first end of the arms, and by a predetermined movement of the device along a direction corresponding to its longitudinal axis in a brain tissue.

According to preferred embodiments, the probe device for interfacing with neurons in a brain tissue comprises an at least partially, or completely rolled up, processed, and patterned flexible 2D substrate.

This provides a very elegant and low-cost production of the neural probe.

According to preferred embodiments, the arms are patterned in the 2D substrate such that they are arranged in a direction generally parallel to the longitudinal axis.

According to preferred embodiments, the neuron interfacing device has a tip portion for insertion of the device into the brain tissue, and the arms are pointing away from the tip portion in the first configuration of the neuron interfacing devices (corresponding to a first configuration of the arms).

According to alternative preferred embodiments, the neuron interfacing device has a tip portion for insertion of the device into the brain tissue, and the arms are pointing towards the tip portion in the first configuration of the neuron interfacing devices (corresponding to a first configuration of the arms).

According to preferred embodiments, the arms are patterned in the 2D substrate such that they are arranged in a direction generally perpendicular to the longitudinal axis, and wherein the arms are wound or rolled up around the longitudinal axis.

In a second aspect of the present disclosure, a composite probe device for interfacing with neurons in a brain tissue is disclosed, comprising a tubular guiding structure for being inserted in a brain tissue and comprising a longitudinal inner bore for receiving and guiding a device according to any of the embodiments of the first aspect, the tubular structure comprising a set of openings having a predetermined configuration being arranged and adapted for guiding the set of neuron interfacing devices towards the predetermined regular grid configuration.

This provides the advantage that the neural probe can be inserted into the brain in a more controlled manner, also reducing the potential damage to the brain tissue.

According to preferred embodiments, the composite device is adapted for guiding a neuron interfacing device comprising a support structure comprises a longitudinal central portion and a set of at least 1 (e.g.1) (or at least 2 (e.g. 2), or at least 3 (e.g. 3), or at least 4 (e.g. 4)) flexible arm(s), each arm generally extending from a respective first end at a central portion of said support structure to a second end being a freestanding distal end, and each arm comprising at least one neuron interfacing device, the arms being adapted and arranged for positioning said neuron interfacing devices in said predetermined regular grid configuration by bending radially outwards according to a predetermined extent, when in said second configuration, and the openings are arranged and adapted for allowing respective arms to penetrate respective openings of the set of openings, when the device is moved along (or in a direction of the axis of) the inner bore.

According to preferred embodiments, the arms are patterned in the 2D substrate such that they are arranged in a direction generally perpendicular to the longitudinal axis, and wherein the arms are wound or rolled up around the longitudinal axis, and the openings are arranged and adapted for allowing respective arms to penetrate respective openings of the set of openings, when the device is rotated around its axis in the bore.

According to preferred embodiments, wherein each of the arms comprises at least 1 neuron interfacing device such as an electrode. According to further preferred embodiments, adjacent or below to some electrodes, adjacent or below to a plurality of electrodes, or adjacent or below each electrode, a light source / LED is provided. The distance between a light source or LED and the respective electrode is preferably smaller than 500 microns. When a light source is provided on below of an electrode, the electrode is preferably embodied as a transparent electrode.

According to preferred embodiments, the neuron interfacing devices of at least one arm are arranged at opposite sides of the arm, such as for instance at opposed sides of a planar substrate out of which the neuron interfacing device is formed. According to preferred embodiments, wherein the arms are patterned into a flexible substrate, one or more neuron interfacing electrodes or neuron interfacing devices are provided on one side of the substrate, while one or more neuron interfacing electrodes or neuron interfacing devices are provided on the other side of the substrate.

In a third aspect of the present disclosure, the use of a probe device according to any of the embodiments of the first aspect or of the second aspect has been disclosed, wherein the neuron interfacing devices are electrodes, for identifying individual neurons in brain tissue.

In a fourth aspect of the present disclosure, a method has been disclosed for determining individual neuron positions in brain tissue, the method comprising implanting a probe device according to any of the embodiments of the first or second aspect into the brain tissue, comprising electrodes as neuron interfacing devices, and reading out signals from the set of electrodes.

According to preferred embodiments, the method further comprises performing a triangulation method for determining specific neuron positions based on the read out of neural signals, e.g. spikes or spike signals, from the set of electrodes. An example of such a method is disclosed in Boussard et.al. (DOI: 10.1101/2021.11.05.467503).

In a fifth aspect of the present disclosure, a method for measuring neuron activity in brain tissue is disclosed, comprising implanting a probe device according to any of the embodiments of the first or second aspect into the brain tissue, comprising electrodes as neuron interfacing devices, and reading out signals from the set of neuron interfacing devices.

In a sixth aspect of the present disclosure, method for stimulating neurons in brain tissue is disclosed, comprising implanting a probe device according to any of the embodiments of the first aspect or a composite device or set of devices according to any of the embodiments of the second aspect into the brain tissue, comprising electrodes as neuron interfacing devices, and providing electrical stimulation signals to the set of electrodes. Preferably, the electrical stimulation signals are provided based on measurement signals obtained by a method according to the fifth aspect. According to preferred embodiments, the stimulation signals for a predetermined neuron can be provided by a pair or a plurality of neighboring electrodes for that neuron. Preferably, but not necessarily, the neighboring electrodes used for stimulation of that neuron are the same or are selected from the electrodes used for the triangulation method used in the fourth aspect.

In a seventh aspect of the present disclosure, a method for manufacturing a probe device for interfacing with neurons in a brain tissue according to any of the embodiments of the first or second aspect, comprising:
- providing a flexible, biocompatible planar (2D) substrate;
- patterning the planar substrate such as to define a set of at least 1 (e.g.1) (or at least 2 (e.g. 2), or at least 3 (e.g. 3), or at least 4 (e.g. 4)) flexible arms;
- processing the planar substrate such as to provide at least one electrically connected neuron interfacing devices, preferably electrodes, for each flexible arm;
- at least partially rolling up the planar substrate into a tubular configuration.

Features and advantages disclosed for one of the above aspects of the present disclosure are hereby also implicitly disclosed for the other aspects, mutatis mutandis, as the skilled person will recognize.

### Brief description of the drawings

The disclosure will be further elucidated by means of the following description and the appended figures.
Fig. 1 is a graph illustrating simulation results of the overlapping monitoring volume of 4 neighboring electrodes (located in a 3D space) for different spatial configurations of neuron interfacing electrodes.
Fig. 2 includes a comparison of Fig. 1 with similar graphs for different values of electrode sensing radius.
Fig. 3 (a) to (e) illustrate the definition of electrode pitch for respective regular grid configurations.
Fig. 3 (f) and (g) illustrate overlapping measurement fields of electrodes.
Fig. 4 (a) to (d) illustrate electrode configurations for neural probes according to embodiments of the present disclosure.
Fig. 5 (a) and (b) illustrate a first preferred embodiment of the present disclosure, which is a composite device comprising a probe device and a tubular guiding structure for the probe device.
Fig. 6 illustrates a second preferred embodiment of a probe device according to the present disclosure, which can preferably be combined with a guiding structure as described for the first embodiment. Further details thereof are illustrated in Fig. 8.
Fig. 7 illustrates a third preferred embodiment of a probe device according to the present disclosure, which can preferably be combined with a guiding structure as described for the first embodiment.
Fig. 9 illustrates a fourth preferred embodiment of a probe device according to the present disclosure, which can preferably be combined with a guiding structure as described for the first embodiment.

### Detailed description of preferred embodiments

The present disclosure will be described with respect to particular embodiments and with reference to certain drawings, but the disclosure is not limited thereto but only by the claims. The drawings described are only schematic and are non-limiting. In the drawings, the size of some of the elements may be exaggerated and not drawn to scale for illustrative purposes. The dimensions and the relative dimensions do not necessarily correspond to actual reductions to practice of the disclosure.

Furthermore, the terms first, second, third and the like in the description and in the claims, are used for distinguishing between similar elements and not necessarily for describing a sequential or chronological order.

The various embodiments, although referred to as "preferred" are to be construed as examples in which the disclosure may be implemented rather than as limiting the scope of the disclosure.

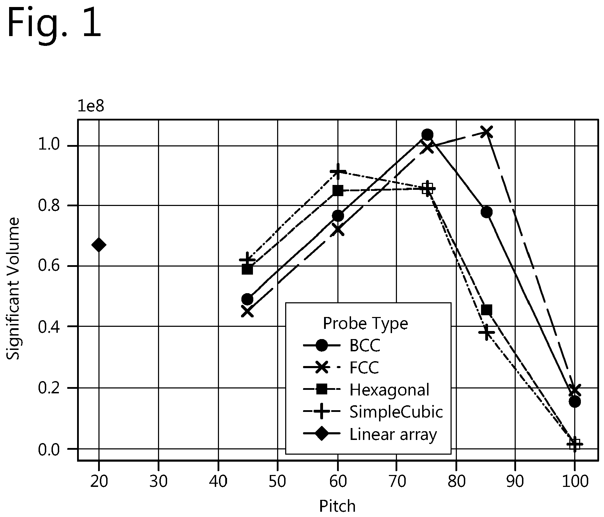

Fig. 1 is a graph illustrating simulation results for different spatial configurations of neuron interfacing electrodes.

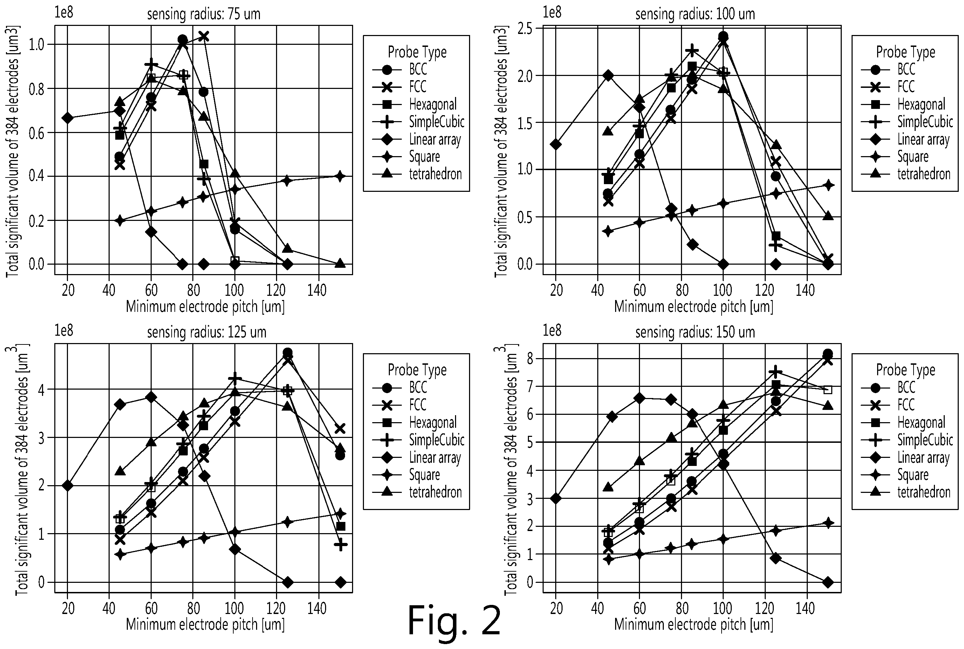

The significant volume (y-axis, in square micron (micron³)) is plotted as a function of the pitch (x-axis, in microns) between neuron interfacing electrodes, for different spatial configurations in the form of regular 3D-grid structures and for a linear array of electrodes (1D). The significant volume is the overlapping monitoring volume of 4 neighboring electrodes that cover a 3D volume, for the respective regular grid configurations. It has been assumed that one electrode can sense activity within a radius of 75 µm (micron). The graph shows which spatial electrode configuration (BCC, FCC, etc.) covers the biggest volume (using a total of 384 electrodes) for an increasing electrode pitch. However, for the increasing electrode pitch it is important that this concerns 4 neighboring electrodes that can sample a 3D volume. That means that only 2 of the 4 electrodes can be located on the same axis. A minimum of 4 electrodes has been used because there are at least 4 electrodes required to define the location of the neuron in a 3D volume. The graph shows a general trend that once the electrode pitch is too small there are too many overlapping and redundant volumes so the overall volume is limited. Once the electrode pitch becomes too big the amount of overlapping is too small, so it is not possible to locate the neuron within that specific spatial configuration. It can be concluded that a linear configuration is the least efficient configuration to sample a volume. Together with fig 2, the FCC and BCC configurations are capable to sample the biggest volume for a specific electrode pitch taking into account that the electrodes can sense with a radius of 75µm-150µm. The simple cubic, tetrahedron or hexagonal grid then capture the 3^{rd}-5^{th} volume for a specific electrode pitch with electrode sensing radius of 75µm-150µm. Finally, electrodes configurated in 2D, in electrode pitch of 45µm-15µm and electrode sensing radius of 75µm-150µm, gain relatively smaller volume than any of the 3D grid configurations.

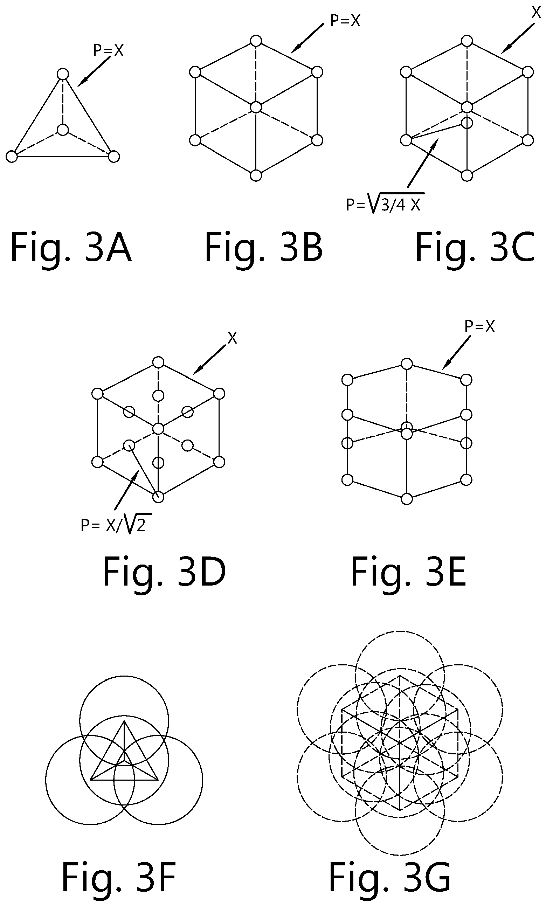

Representations of the different spatial configurations are illustrated in fig 3. It also shows the definition of electrode pitch P per configuration (i.e. is the minimal electrode distance for the respective predetermined regular 3D grid configuration), as a function of the respective grid constant X. Fig. 3(a) illustrates a tetrahedron configuration with 4 electrodes. Fig. 3(b) illustrates a simple cubic configuration with 8 electrodes. Fig. 3(c) illustrates a body centered cubic (BCC) configuration with 9 electrodes. Fig. 3(d) illustrates a face centered cubic (FCC) configuration with 14 electrodes. Fig. 3 (e) illustrates a hexagonal configuration with 12 electrodes.

Figures 3(f) and (g) illustrates the overlapping fields of electrodes when assuming that a sensing radius of the electrodes is about 2/3 of the electrode pitch P, for a tetrahedron and simple cubic configuration.

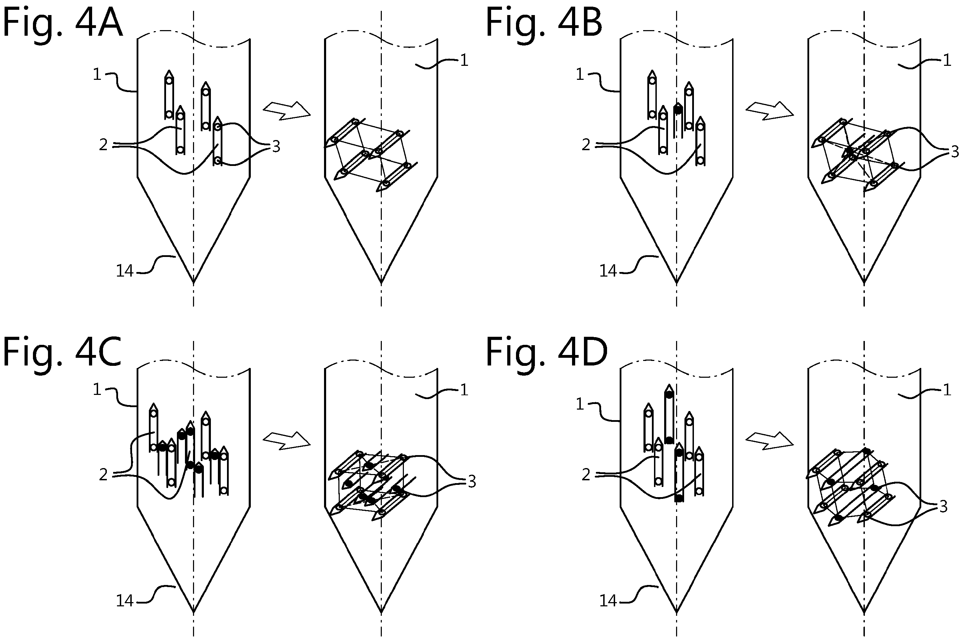

Fig. 4 (a) to (d) illustrate electrode configurations for neural probes 100 according to embodiments of the present disclosure. The neural probes 100 can consist of a support structure 1 or can be composite devices comprising the support structure 1 and a tubular guiding structure 4 (not depicted in Fig. 4 (a) to (d)).

The probes 100 comprise a support structure 1 which is preferably made of a flexible biocompatible material, such as for instance polyimide, parylene, epoxy, PEEK, Ultem, SU-8, PDMS or silicone. It is adapted for interfacing with neurons in a brain tissue such that the material does not damage the cells and the cells do not damage the structural material. It comprises a support structure 1 having a longitudinal axis and set of neuron interfacing electrodes 3 arranged on the support structure 1.

The set of neuron interfacing electrodes 3 is arranged and adapted to be movable between a first, insertion configuration and a second, extended configuration. The first configuration is different from the second configuration. The first configuration (on the left of each pair of associated figures (a), (b), (c), (d)) is adapted for reaching a predetermined target area in the brain while creating a minimal damage to the brain. The second configuration corresponds to a simple cubic (or cubic), face centered cubic, body centered cubic, hexagonal, or tetrahedron grid configuration.

The support structure 1 defines a longitudinal probe shape defining a circumferential wall comprising a cylinder mantle portion describing an angular section, between e.g. 180° and 360° of a cylinder surface, and the neuron interfacing electrodes 3 are abutting said cylinder mantle portion, when in the first configuration. The support structure 1 comprises a longitudinal central portion and a set of flexible arms 2, each arm 2 generally extending from a respective first end at a central portion of the support structure 1 to a second end being a freestanding distal end, and each arm 2 comprising at least one electrode 3. The arms 2 are adapted and arranged for positioning the electrodes 3 in the predetermined regular grid configuration by bending radially outwards according to a predetermined extent, when moving towards and ending in the second configuration of the electrodes 3 (corresponding to a second configuration of the arms; on the right of each pair of associated figures). The arms have a pointed distal end for insertion into brain tissue.

In Fig. 4(a) the second configuration of the electrodes corresponds to a cubic configuration.

In Fig. 4(b) the second configuration of the electrodes corresponds to a body centered configuration.

In Fig. 4(c) the second configuration of the electrodes corresponds to a face centered configuration.

In Fig. 4(d) the second configuration of the electrodes corresponds to a hexagonal grid configuration.

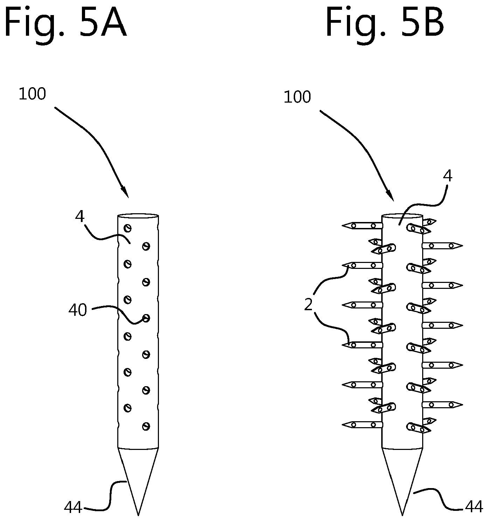

Preferably, as illustrated in Fig. 5, the support structure 1 is part of a composite device or probe 100 (1,4), comprising a tubular guiding structure 4 for being inserted in brain tissue and comprising a longitudinal inner bore or cylindrical volume for receiving and guiding the support structure 1. The tubular guiding structure comprises a pointy, sharp insertion tip 44 at a first end for penetrating the brain tissue. The support structure 1 can be positioned in the tubular guiding structure 4 when the guiding structure is inserted in the brain. Alternatively, the support structure 1 may be received by the guiding structure 4 after the guiding structure has been inserted in the brain. The material of the tubular guiding structure should be stiff enough to penetrate the brain tissue and can be made of biocompatible polymer material or a rigid material using micro-fabrication techniques. The tubular structure 4 comprises a set of openings 40 having a predetermined configuration being arranged and adapted for guiding the set arms 2 and thus neuron interfacing electrodes 3 towards the predetermined regular grid configuration. The openings 40 are arranged and adapted for allowing respective arms 2 to penetrate respective openings of the set of openings 40, when the support structure 1 is moved relative to and inside the inner bore of the guiding structure 4, for instance by sliding it along the direction of the bore (or axis of cylindrical volume) towards or away from the insertion tip 44 of the tubular structure 4, or by rotating the support structure 1 about its axis within the bore or cylindrical volume.

The support structures 1 or probes 100 (in the absence of a tubular guiding structure 4) are manufactured by partially, or completely rolling up a processed and patterned flexible 2D substrate 10. The arms 2 are patterned in the 2D substrate in a predetermined manner, before the substrate 10 is rolled up. The arms 2 are extend in a direction generally parallel to the longitudinal axis, in the first configuration of the electrodes, corresponding to a first configuration of the arms 2.

In the depicted embodiments of Fig. 4 (a) to (d), the neural probe 100 (or support structure 1 in the absence of a tubular guiding structure 4) has a tip portion 14 for insertion of the device into the brain tissue and the arms 2 are pointing away from the tip portion 14, in the first configuration of the electrodes (and arms). A slight withdrawal of the probe in a direction outwardly from the brain, will open up the arms and cause them to penetrate the brain tissue radially outwardly.

The embodiments of Fig. 4 (a) to (d) can for instance be used independently or in configuration with a tubular guiding structure 4 as explained in relation with Fig. 5, whereby in the latter case the arms point away from the tip portion 44 in their first configuration (corresponding to the first configuration of the electrodes).

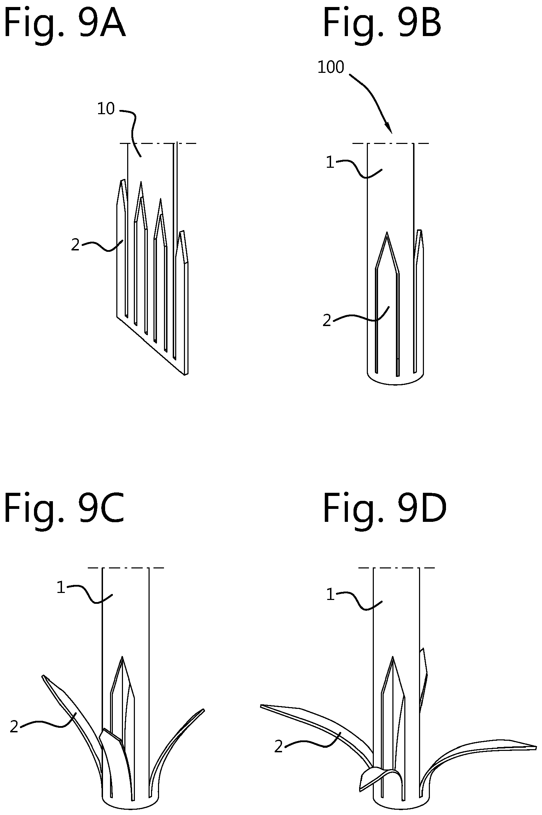

Figures 9 (a) to (d) illustrate embodiments of support structures 1 similar to those of Fig. 4 (a) to (d) and which can be used in combination with a tubular guiding structure 4. The support structure of Fig. 9(a) to (d) does not comprise a pointed tip portion 14.

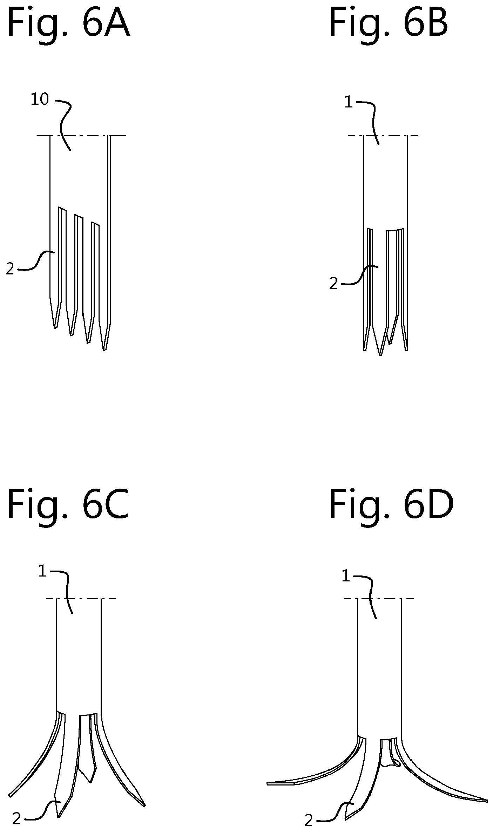

Alternatively, illustrated in Fig. 6 (a) to (d), the support structure 1 is used in combination with a tubular guiding structure 4 having a tip portion 44 at its lower end (not depicted), the arms 2 can be pointing towards a tip portion 44 of the tubular guiding structure 44.

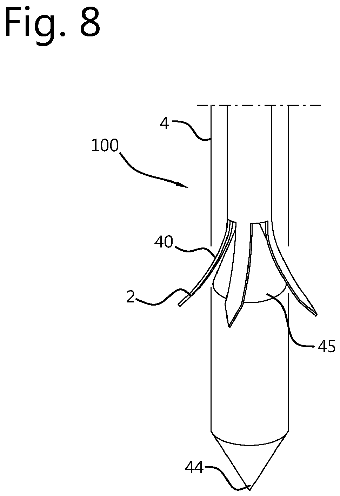

When moving the support structure 1 axially along the longitudinal axis of the bore (away or towards the tip portion 44, depending on the embodiments), the arms 2 encounter the respective openings 40 in the tubular guiding structure 4, and extend through it into the brain tissue, thereby exposing the electrodes 3 to the brain tissue. Alternatively, or in combination therewith, the arms 2 can also deploy due to guides 45 arranged inside the tubular guiding structure 4, as shown in Fig 8. The guide 45 can for instance comprise a rounded surface suitable for guiding the arms towards the respective openings in the tubular guiding structure 4.

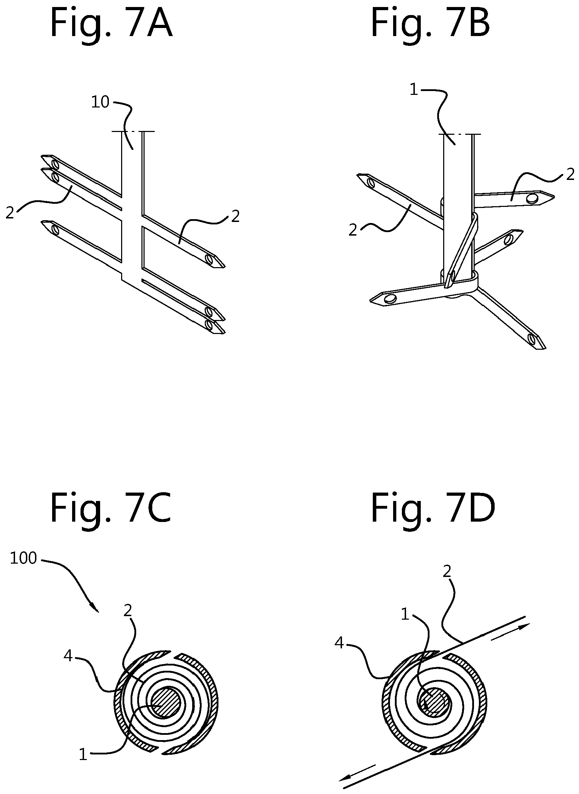

In an embodiment depicted in Fig. 7 (a) to (d), the arms are patterned in the 2D substrate such that they are initially arranged in a direction generally perpendicular to the longitudinal axis of the probe or, more preferably, composite probe, and the arms 2 are wound along the longitudinal axis. When unwinding the arms 2 by rotating the support structure 1 about its longitudinal axis in the bore, the arms 2 encounter the respective openings 40 in the tubular guiding structure 4, and extend through it into the brain tissue, thereby exposing the electrodes 3 to the brain tissue.

For all of the previous embodiments, each of the arms comprises at least 1 electrode 3. According to preferred embodiments, each of the arms comprises 1 or 2 electrodes 3.

In certain embodiments, wherein at least one arm 2 of the plurality of arms 2 comprises at least 2 electrodes 3, electrodes 3 can be provided on opposite sides of the arms 2, corresponding to opposite sides of the substrate 10.

For all of embodiments, the electrodes 3 or other neuron interfacing devices such as optical interaction devices are connected to readout or stimulation circuitry by means of suitable electrical connections, as it is known in the art.

It will be appreciated by the skilled person that:
- the disclosed neural implants or probes 100 can efficiently be used for identifying individual neurons in brain tissue, to measure neuron activity, especially of a predetermined, e.g. identified neuron, and to stimulate one or more predetermined, e.g. identified neurons in brain tissue;
- the neural probe 100 can be implanted into brain tissue, and signals can be read out from the electrodes 3;
- a triangulation method can be used for determining specific neuron positions based on read out signals from the set of electrodes of the disclosed neural implants. An example of a triangulation method that can be used is described by Boussard et.al. (DOI:10.1101/2021.11.05.467503).

The disclosed embodiments of a probe device for interfacing with neurons in a brain tissue can be manufactured by a method comprising the following steps;
- providing a flexible, biocompatible planar (2D) substrate 10; for instance, the material can be applied by spin coating or other MEMS deposition techniques onto a carrier substrate. The substrate can be photosensitive or non-photosensitive for patterning purposes.
- patterning the planar substrate 10 such as to define a set of at least 2, e.g. 2, 3 or 4 flexible arms 2, e.g. by using micromachining techniques such as dry etching techniques or wet etching techniques or by photolithography of the material itself;
- processing the planar substrate 10 such as to provide at least one electrically connected neuron interfacing electrode 3 for each flexible arm 2, for instance by using metal deposition techniques such as PECVD or sputter coating techniques, where the metal can be patterned using a lift-off technique or using dry or wet etching techniques, or e.g. by plating. For very small feature sizes, of the nanometer size, e.g. smaller than e.g. 5 nm, or smaller than 3 nm, focused ion beam techniques can for instance be used;
- rolling up the planar substrate into a tubular configuration, for instance by using molds and micromanipulators.

Some advantages of embodiments and aspects of the present disclosure are the following:
- the disclosed probes 100 can identify more neurons than probes having the same number of microelectrodes used in standard linear electrode arrays (by a factor of 10);
- reduced tissue damage in case of the composite device; the tissue damage is largely limited to the damage induced by the tubular guiding structure 4, while the recordings would be made from the electrodes on the thin flexible polymer arms 2 (the substrate and polymer arms have a typical thickness between 1 and 30 microns);
- the configurations of the electrodes 3 is such that they gain most of the 3D spatial features of spike waveforms allowing better neuron differentiation and localization compared to conventional linear electrode arrays;
- the disclosed probes 100 having the claimed electrode configuration can also reach deep brain areas located deeper than 10mm from the brain surface. Something which is not possible using stacked 2D probe arrays or a matrix of 1D linear probes of the prior art.

What could be claimed:
1. A probe device (100) for interfacing with cells, preferably neurons in brain tissue, comprising a support structure (1) having a longitudinal axis and set of cell or neuron interfacing electrodes (3) arranged on the support structure (1), wherein the set of cell or neuron interfacing electrodes (3) is arranged and adapted to be movable between a first, insertion configuration and a second extended configuration, the first configuration being different from the second configuration, wherein the first configuration is adapted for reaching a predetermined target area in the brain while creating a minimal damage to the brain and wherein the second configuration corresponds to a predetermined regular 3-dimensional grid configuration, the predetermined regular 3-dimensional grid configuration corresponding to a cubic, face centered cubic (fcc), body centered cubic (bcc), hexagonal or tetrahedron grid configuration.
2. A probe device (100) according to item 1, wherein the neuron interfacing electrode comprises a sensing radius within the range of 75 micron to 150 micron, and wherein an electrode pitch of the neuron interfacing electrode is within the range of 45 micron to 150 micron.
3. A probe device (100) according to item 1 or 2, wherein the predetermined regular 3-dimensional grid configuration corresponds to a cubic, face centered cubic (fcc), body centered cubic (bcc) configuration.
4. A device according to any of the previous items, wherein the support structure comprises a flexible or stretchable material such as a flexible biocompatible polymer.
5. A device according to any of the previous items, wherein the support structure generally defines a longitudinal probe shape defining a circumferential wall comprising a cylinder mantle portion describing an angular section of a cylinder surface, and wherein the neuron interfacing electrodes are abutting the cylinder mantle portion, when in the first configuration.
6. A device according to any of the previous items, wherein said support structure comprises a longitudinal central portion and a set of at least 4 flexible arms (2), each arm generally extending from a respective first end at a central portion of said support structure to a second end being a freestanding distal end, and each arm (2) comprising at least one electrode, wherein said arms are adapted and arranged for positioning said electrodes in said predetermined regular grid configuration by bending radially outwards according to a predetermined extent, when in said second configuration.
7. A device according to item 6, wherein the predetermined extent is determined by a predetermined internal stress in the arms causing a radially outward bias of the distal end with respect to the first end of the arms, and by a predetermined movement of the device along a direction corresponding to its longitudinal axis in a brain tissue.
8. A device according to any of the previous items 6 or 7, comprising an at least partially, or completely rolled up, processed, and patterned flexible 2D substrate (10).
9. A device according to item 8, wherein the arms are patterned in the 2D substrate (10) such that they are arranged in a direction generally parallel to the longitudinal axis.
10. A device according to item 9, wherein the device has a tip portion (14) for insertion of the device into the brain tissue, and wherein the arms (2) are pointing away from the tip portion (14) in the first configuration of the electrodes (3).
11. A device according to item 9, wherein the device has a tip portion (14) for insertion of the device into the brain tissue, and wherein the arms (2) are pointing towards the tip portion (14) in the first configuration of the electrodes.
12. A device according to item 8 or 9, wherein the arms (2) are patterned in the 2D substrate (10) such that they are arranged in a direction generally perpendicular to the longitudinal axis, and wherein the arms (2) are wound along the longitudinal axis.
13. A device according to any of the previous items 6 to 12, wherein the arms comprise at least 1 electrode (3).
14. A device according to item 13, comprising at least one arm having at least two electrodes (3), and wherein the electrodes of the at least one arm (2) are arranged at opposite sides of the arm.
15. A composite device or set of devices, comprising a tubular guiding structure (4) for being inserted in a brain tissue and comprising a longitudinal inner bore for receiving and guiding a device according to any of the previous items, the tubular structure (4) comprising a set of openings (40) having a predetermined configuration being arranged and adapted for guiding the set of neuron interfacing electrodes (3) towards the predetermined regular grid configuration.
16. A composite device or set of devices according to item 15, for guiding a device according to any of items 1 to 14, wherein the openings (40) are arranged and adapted for allowing respective arms (2) to penetrate respective openings of the set of openings (40), when the device is moved along the inner bore.
17. A composite device or set of devices according to item 15, for guiding a device according to any of items 1 to 14, wherein the openings (40) are arranged and adapted for allowing respective arms (2) to penetrate respective openings of the set of openings, when the device is rotated around its axis in the bore.
18. The use of a device according to any of item 1 to 14 or a composite device or set of devices according to any of items 15 to 17, for identifying individual neurons in brain tissue.
19. A method for determining individual neuron positions in brain tissue, comprising implanting a device according to any of item 1 to 14 or a composite device or set of devices according to any of items 15 to 17 into the brain tissue, and reading out signals from the set of electrodes (3).
20. A method according to item 19, further comprising performing a triangulation method for determining specific neuron positions based on the read-out signals from the set of electrodes (3).
21. A method for measuring neuron activity in brain tissue, comprising implanting a device according to any of item 1 to 14 or a composite device or set of devices according to item 15 to 17 into the brain tissue, and reading out signals from the set of electrodes (3).
22. A method for stimulating neurons in brain tissue, comprising implanting a device according to any of item 1 to 14 or a composite device or set of devices according to any of items 15 to 17 into the brain tissue, and providing, preferably electrical, stimulation signals to the set of electrodes (3).
23. A method for manufacturing a probe device 100 for interfacing with neurons in a brain tissue, comprising:
   - Providing a flexible, biocompatible planar (2D) substrate (10);
   - Patterning the planar substrate (10) such as to define a set of at least 1 (e.g.1) (or at least 2 (e.g. 2), or at least 3 (e.g. 3), or at least 4 (e.g. 4)) flexible arms (2);
   - Processing the planar substrate such as to provide at least one electrically connected neuron interfacing electrode (3) for each flexible arm (2);
   - Rolling up the planar substrate into a tubular configuration.

## Claims

1. A probe (100) device for interfacing with cells, preferably neurons in brain tissue, comprising a support structure (1) having a longitudinal axis and set of cell or neuron interfacing electrodes (3) arranged on said support structure (1), wherein said set of cell or neuron interfacing electrodes (3) is arranged and adapted to be movable between a first, insertion configuration and a second extended configuration, said first configuration being different from the second configuration, wherein said first configuration is preferably adapted for reaching a predetermined target area in said brain while creating a minimal damage to said brain and wherein said second configuration corresponds to a predetermined regular 3-dimensional grid configuration, said predetermined regular 3-dimensional grid configuration corresponding to a cubic, face centered cubic (fcc), body centered cubic (bcc), hexagonal or tetrahedron grid configuration.

2. A probe (100) device according to claim 1, wherein said neuron interfacing electrode comprises a sensing radius within the range of 75 micron to 150 micron, and wherein an electrode pitch of said neuron interfacing electrode (3) is within the range of 45 micron to 150 micron.

3. A device according to any of the previous claims, wherein said support structure (1) comprises a flexible or stretchable material such as a flexible or stretchable biocompatible polymer.

4. A device according to any of the previous claims, wherein said support structure (1) generally defines a longitudinal probe shape defining a circumferential wall comprising a cylinder mantle portion describing an angular section of a cylinder surface, and wherein said neuron interfacing electrodes are abutting said cylinder mantle portion, when in said first configuration.

5. A device according to any of the previous claims, wherein said support structure (1) comprises a longitudinal central portion and a set of at least 1 flexible arm (2), each arm generally extending from a respective first end at a central portion of said support structure to a second end being a freestanding distal end, and each arm (2) comprising at least one electrode (3), wherein said arms are adapted and arranged for positioning said electrodes (3) in said predetermined regular grid configuration by bending radially outwards according to a predetermined extent, when in said second configuration.

6. A device according to claim 5, comprising an at least partially, or completely rolled up, processed and patterned flexible 2D substrate (10).

7. A device according to claim 5 or 6, wherein said arms (2) are patterned in said 2D substrate (10) such that they are arranged in a direction generally parallel to said longitudinal axis.

8. A device according to claim 7, wherein said arms (2) are patterned in said 2D substrate (10) such that they are arranged in a direction generally perpendicular to said longitudinal axis, and wherein said arms (2) are wound along said longitudinal axis.

9. A composite device or set of devices, comprising a tubular guiding structure (4) for being inserted in a brain tissue and comprising a longitudinal inner bore for receiving and guiding a device according to any of the previous claims, said tubular guiding structure (4) comprising a set of openings (40) having a predetermined configuration being arranged and adapted for guiding said set of neuron interfacing electrodes (3) towards said predetermined regular grid configuration.

10. A composite device or set of devices according to claim 9, for guiding a device according to any of claims 1 to 8, wherein said openings (40) are arranged and adapted for allowing respective arms (2) to penetrate respective openings of said set of openings (40), when said device is moved along said inner bore.

11. A composite device or set of devices according to claim 9, for guiding a device according to any of claims 1 to 8, wherein said openings (40) are arranged and adapted for allowing respective arms (2) to penetrate respective openings of said set of openings (40), when said device is rotated around its axis in said bore.

12. A method for determining individual neuron positions in brain tissue, comprising implanting a device according to any of claim 1 to 8 or a composite device or set of devices according to any of claims 9 to 11 into said brain tissue, and reading out signals from said set of electrodes.

13. A method for measuring neuron activity in brain tissue, comprising implanting a device according to any of claim 1 to 8 or a composite device or set of devices according to claim 9 to 11 into said brain tissue, and reading out signals from said set of electrodes.

14. A method for stimulating neurons in brain tissue, comprising implanting a device according to any of claim 1 to 8 or a composite device or set of devices according to any of claims 9 to 11 into said brain tissue, and providing, preferably electrical, stimulation signals to said set of electrodes.

15. A method for manufacturing a probe device (100) for interfacing with neurons in a brain tissue, comprising:
- Providing a flexible, biocompatible planar (2D) substrate (10);
- Patterning said planar substrate (10) such as to define a set of at least 1 (e.g.1) (or at least 2 (e.g. 2), or at least 3 (e.g. 3), or at least 4 (e.g. 4)) flexible arm(s) (2);
- Processing said planar substrate (10) such as to provide at least one electrically connected neuron interfacing electrode (3) for each flexible arm (2);
- Rolling up said planar substrate (10) into a tubular configuration.
